# EUROPEAN PATENT SPECIFICATION

(11) **EP 1 123 396 B1**
(45) Date of publication and mention of the grant of the patent: **08.03.2006**
(21) Application number: 99960139.6
(22) Date of filing: 19.10.1999
(51) Int. Cl.: C12N 15/12, A61K 48/00, C12M 1/00

(54) **MINIMAL PROMOTERS AND USES THEREOF**
MINIMALE PROMOTOREN UND IHRE VERWENDUNG
PROMOTEURS TRONQUES ET UTILISATIONS ASSOCIEES

(30) Priority: 19.10.1998 US 104871 P
(43) Date of publication of application: 16.08.2001
(73) Proprietor: Powderject Vaccines, Inc., Madison, Wisconsin 53711 (US)
(72) Inventor: FULLER, James, T., Madison, WI 53711 (US)
(74) Representative: Woods, Geoffrey Corlett
(86) International application number: PCT/US1999/024694
(87) International publication number: WO 2000/023592

(56) References cited:
- WO-A-89/01036
- WO-A-98/20041
- WO-A-98/37185
- MOHAMMADI ET AL: "EFFICIENT TRANSGENE REGULATION FROM A SINGLE TETRACYCLINE-CONTROLLED POSITIVE FEEDBACK REGULAORY SYSTEM" GENE THERAPY, vol. 5, January 1998 (1998-01), pages 76-84, XP000891454
- HORMANN ET AL: "RAPID RETROVIRAL DELIVERY OF TETRACYCLINE-INDUCIBLE GENES IN A SINGLE AUTOREGULATORY CASSETTE" PROCEEDINGS OF THE NATIONAL ACADEMY OF SCIENCES, USA, vol. 93, 1996, pages 5185-5190, XP002136742
- BOEHM WALTRAUD ET AL: "DNA vector constructs that prime hepatitis B surface antigen-specific cytotoxic T lymphocyte and antibody responses in mice after intramuscular injection." JOURNAL OF IMMUNOLOGICAL METHODS 1996, vol. 193, no. 1, 1996, pages 29-40, XP002136743 ISSN: 0022-1759

## Description

### Technical Field

The invention relates to the general fields of molecular biology and immunology, and generally relates to reagents useful in nucleic acid immunization techniques. More specifically, the invention relates to truncated forms of transcriptional promoter elements, nucleic acid molecules containing such promoter elements, and to the use of reagents containing such nucleic acid molecules for nucleic acid immunization .

### Background

Techniques for the injection of DNA and mRNA into mammalian tissue for the purposes of immunization against an expression product have been described in the art. See, e.g., European Patent Specification EP 0 500 799 and U.S. Patent No. 5,589,466. The techniques, termed "nucleic acid immunization" herein, have been shown to elicit both humoral and cell-mediated immune responses. For example, sera from mice immunized with a DNA construct encoding the envelope glycoprotein, gp160, were shown to react with recombinant gp160 in immunoassays, and lymphocytes from the injected mice were shown to proliferate in response to recombinant gp120. Wang et al. (1993) *Proc. Natl. Acad. Sci. USA* 90:4156-4160. Similarly, mice immunized with a human growth hormone (hGH) gene demonstrated an antibody-based immune response. Tang et al. (1992) *Nature* 356:152-154. Intramuscular injection of DNA encoding influenza nucleoprotein driven by a mammalian promoter has been shown to elicit a CD8+ CTL response that can protect mice against subsequent lethal challenge with virus. Ulmer et al. (1993) *Science* 259:1745-1749. Immunohistochemical studies of the injection site revealed that the DNA was taken up by myeloblasts, and cytoplasmic production of viral protein could be demonstrated for at least 6 months.

It has been a major aim of artisans practising in the fields of gene therapy and nucleic acid immunization to identify and design expression systems that provide as high a level of production as possible for a gene or sequence of interest that has been delivered into a host cell. High level production is seen as being a necessary prerequisite for any expression system used in gene therapy (to provide for sufficient levels of a therapeutic gene product) or nucleic acid immunization (to provide for a sufficient immune response against an encoded antigen product). Several factors are known to affect the level of production attainable from such transfected host cells, including transfection efficiency (e.g., the copy number in the cell) and the efficiency with which the gene or sequence of interest is transcribed and the mRNA translated.

Accordingly, a number of expression systems have been described in the art, each of which typically consists of a vector containing a gene or nucleotide sequence of interest operably linked to expression control sequences which control the expression of the gene or nucleotide sequence. These control sequences include transcriptional promoter sequences and transcriptional start and termination sequences. Transcriptional promoter sequences are generally located just upstream of initiation sites for RNA transcripts, and include a "TATA" box and a "CAAT" box, respectively located about 30 and 80 nucleotides upstream (e.g., at about the -30 and -80 position) relative to the initiation site. Corden et al. (1980) *Science* 209:1406-1414; Chambon, P. (1981) *Ann. Rev. Biochem.* 50:349-383. Commonly used promoters for mammalian cell expression systems include the SV40 early promoter, a CMV promoter such as the CMV immediate early promoter (Chapman et al. (1991) *Nucl. Acids Res.* 19:3979-3986), the mouse mammary tumor virus LTR promoter, the adenovirus major late promoter (Ad MLP), and the herpes simplex virus promoter, among others. Nonviral promoters, such as a promoter derived from the murine metallothionein gene, are also commonly used for such expression systems. These promoter systems are normally selected to provide for the highest level of expression possible for a given sequence.

A number of transcriptional modulator elements that are found more than 110 nucleotides upstream (-110) of various genes have also been commonly used in the art. These upstream sequences are deemed essential for the expression of early genes in simian, murine and human DNA viruses, and are commonly referred to as "enhancers." Enhancers are broadly defined as a cis-acting agent which, when operably linked to a promoter/gene sequence, will dramatically increase transcription of that gene sequence. Enhancers can function from positions that are much further away from a sequence of interest than other expression control elements (e.g., promoters), and can operate when positioned in either orientation relative to the sequence of interest. Banerji et al. (1981) *Cell* 27:299-308, de Filleirs et al. (1981) *Nucleic Acids Research* 9:6251-6264. Enhancers have been identified from a number of viral sources, including polyoma virus, BK virus, cytomegalovirus (CMV), adenovirus, simian virus 40 (SV40), Moloney sarcoma virus, bovine papilloma virus, and Rous sarcoma virus (de Villeirs et al. *supra,* Rosenthal et al. (1983) *Science* 222:749-755, Hearing et al. (1983) *Cell* 33:695-703, Weeks et al. (1983) *Mol. Cell. Biol.* 3:1222-1234, Levinson et al. (1982) *Nature* 295:568-572, and Luciw et al. (1983) *Cell* 33:705-716). These enhancer elements are often coupled with homologous and heterologous promoter sequences to provide enhancer/promoter pairs for use in expression systems. However, probably the most frequently used enhancer/promoter pair used in gene therapy and nucleic acid immunization is the hCMV immediate-early enhancer/promoter pair. See e.g.; U.S. Patent Nos. 5,168,062 and 5,385,839 to Stinksi, and EP Patent Specification 0 323 997 B1. The hCMV enhancer element has also been used without the hCMV promoter element, for example to modulate a heterologous promoter sequence. See e.g., EP Patent Specification 0 173 177 B 1. The hCMV immediate-early enhancer/promoter pair provides for robust expression from a variety of different sequences of interest, and this high level expression is commonly thought of as establishing the "gold standard" for any given expression system.

WO 98/20041 is concerned with a regulatory element present in the endothelial protein C promoter which directs expression of genes in endothelial cells Mohammadi et al., Gene Therapy 5, 76-84, reports transgene regulation by a construct comprising a CMV enhancerless promoter/tetracycline-controlled response element. Hofman et al., PNAS USA 93, 5185-5190, 1996, discloses use of a CMV minimal promoter in a retroviral vector construct, together with a tetracycline control element. WO 98/37185 describes a tetracycline responsive expression vector which utilises a minimal promoter. Bohm et al., J. Immunol. Methods 193, 29-40, 1996, describes DNA vector constructs that prime hepatitis B surface antigen-specific cytotoxic T lymphocyte and antibody responses in mice after intramuscular injection.

### Summary of the invention

It is a primary object of the invention to provide a promoter system which provides a better expression character in mammalian cells, particular *in vivo.* It has now surprisingly been found that the use of a promoter sequence that normally is paired with its homologous enhancer sequence in an expression system will provide for a greatly enhanced immune response against an encoded antigen when the promoter is used in a truncated, enhancer-less form in an expression system. The "enhancerless" promoter sequence is referred to herein as a "minimal promoter". An improved immunological response to a nucleic acid vaccine composition which incorporates the promoter system can be achieved.

Accordingly, the present invention provides use of a nucleic acid construct comprising a coding sequence encoding an antigen and a promoter sequence which is not coupled to its native enhancer sequence and which is operably linked to the coding sequence, for the manufacture of a medicament for use in nucleic acid immunization of a mammalian subject, wherein said antigen is an antigen of a viral, bacterial, parasite or fungal pathogen, or is a tumor-specific antigen or an antigen associated with an autoimmune disease. The invention also provides:
- coated particles suitable for use in particle-mediated nucleic acid immunization, which particles comprise carrier particles coated with a nucleic acid construct comprising a coding sequence encoding an antigen and a promoter sequence which is not coupled to its native enhancer sequence and which is operably linked to the coding sequence, wherein said antigen is an antigen of a viral, bacterial, parasite or fungal pathogen, or is a tumor-specific antigen or an antigen associated with an autoimmune disease and
- a particle acceleration device suitable for particle-mediated nucleic acid immunization, the said device being loaded with coated particles of the invention.

The nucleic acid construct may be present in a vector construct, for example in a plasmid vector. A nucleic acid immunization reagent capable of eliciting an immune response against an antigen of interest is thus provided, the reagent comprising a nucleic acid construct which includes a nucleic acid sequence of interest under the transcriptional control of a minimal promoter sequence. Expression systems can be constructed for use in nucleic acid immunization to provide for a greatly enhanced immune response against an antigen of interest.

These and other objects, aspects, embodiments and advantages of the present invention will readily occur to those of ordinary skill in the art in view of the disclosure herein.

### Brief Description of the Figures

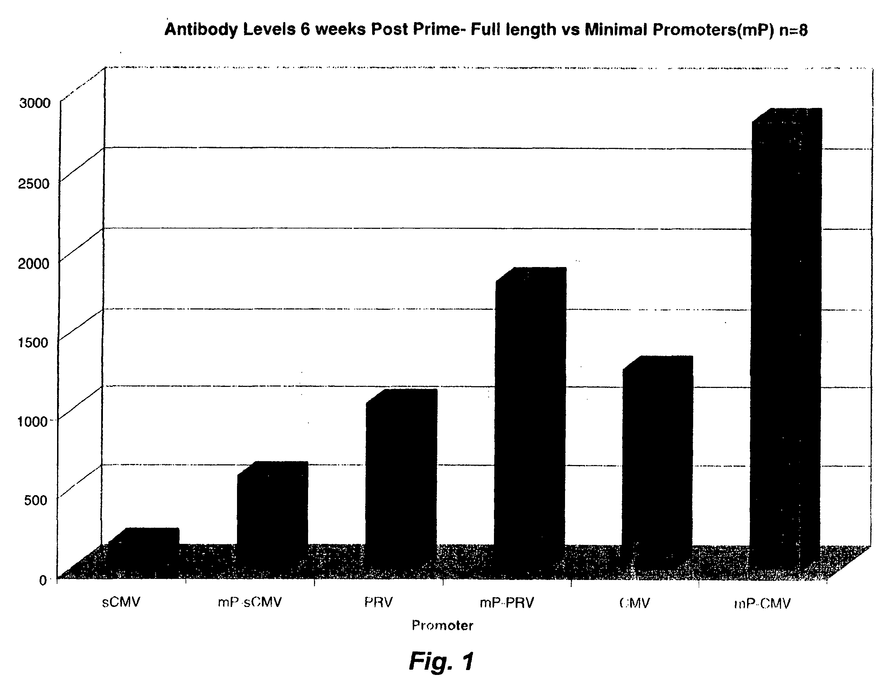

Figure 1 depicts a comparison between the minimal promoter constructs of the present invention and their corresponding enhanced promoter constructs. The *in vivo* performance of the promoter constructs was assessed by measuring antibody production against the antigen of interest in animals receiving nucleic acid immunization with the constructs.

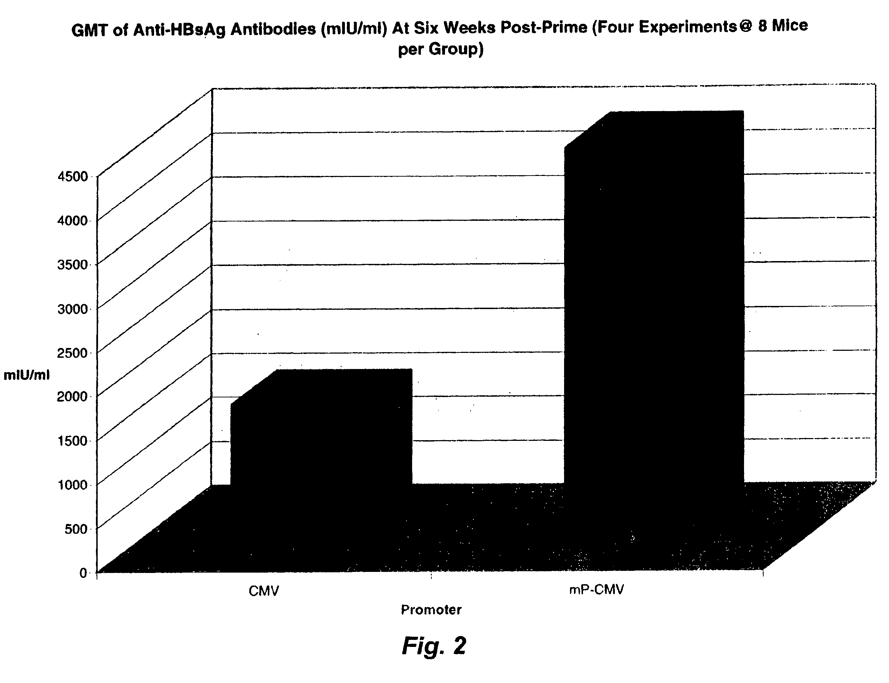

Figure 2 depicts a comparison between a minimal human cytomegalovirus (hCMV) promoter construct produced according to the present invention, and its corresponding, fully enhanced hCMV promoter construct.

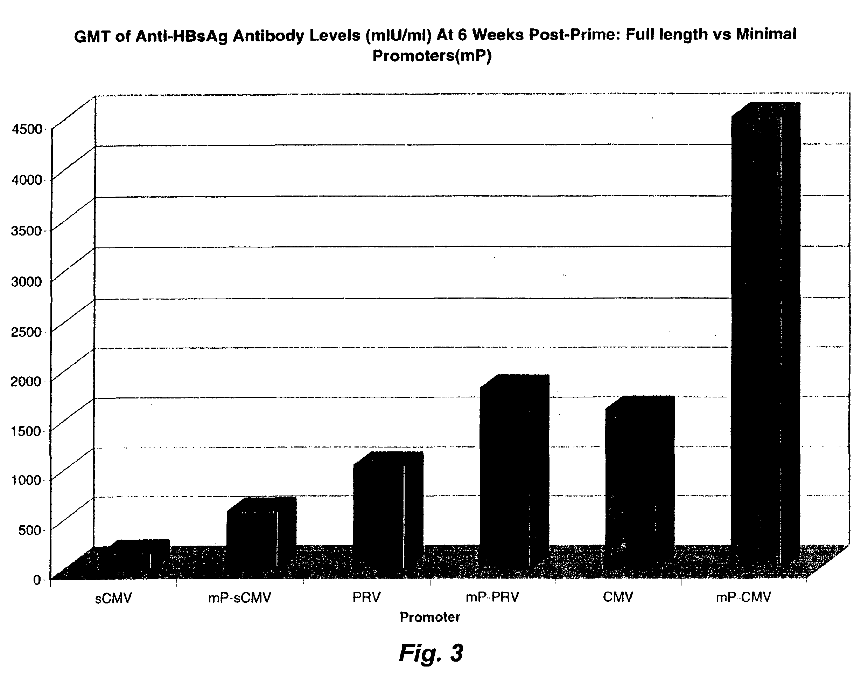

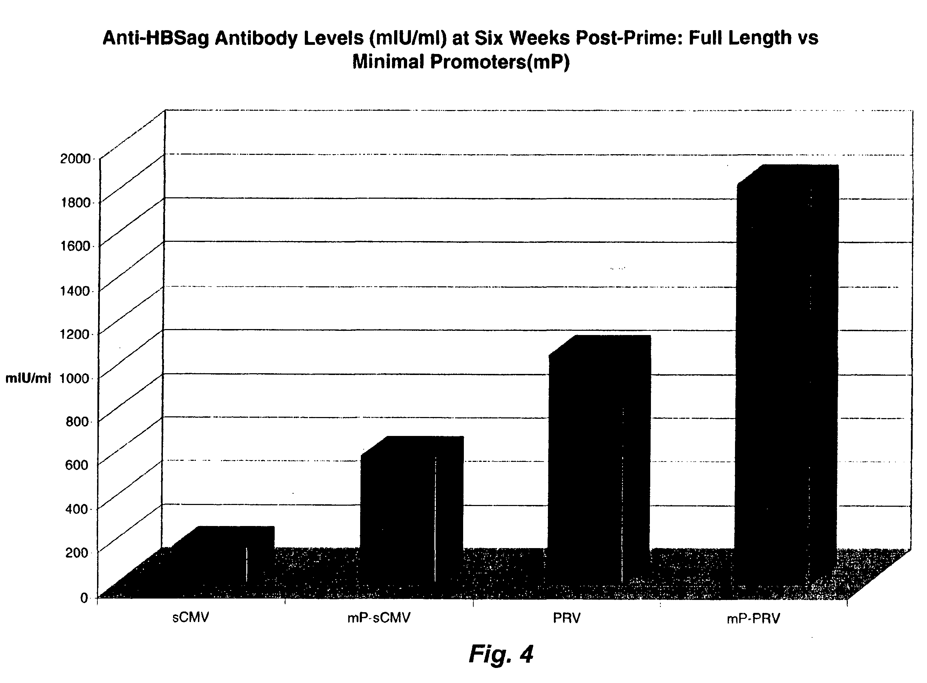

Figures 3 and 4 also depict comparison results obtained from *in vivo* vaccination studies using the minimal promoter constructs of the present invention and their corresponding enhanced promoter constructs. Here again, the *in vivo* performance of the promoter constructs was assessed by measuring antibody production against the antigen of interest in animals receiving nucleic acid immunization with the constructs.

### Detailed Description of the Preferred Embodiments

Before describing the present invention in detail, it is to be understood that this invention is not limited to particularly exemplified molecules or process parameters as such may, of course, vary. It is also to be understood that the terminology used herein is for the purpose of describing particular embodiments of the invention only, and is not intended to be limiting. In addition, the practice of the present invention will employ, unless otherwise indicated, conventional methods of virology, microbiology, molecular biology, recombinant DNA techniques and immunology all of which are within the ordinary skill of the art. Such techniques are explained fully in the literature. See, e.g., Sambrook, et al., *Molecular Cloning: A Laboratory Manual* (2nd Edition, 1989); *DNA Cloning: A Practical Approach,* vol. I & II (D. Glover, ed.); *Oligonucleotide Synthesis* (N. Gait, ed., 1984); *A Practical Guide to Molecular Cloning* (1984); and *Fundamental Virology,* 2nd Edition, vol. I & II (B.N. Fields and D.M. Knipe, eds.).

It must be noted that, as used in this specification and the appended claims, the singular forms "a," "an" and "the" include plural referents unless the content clearly dictates otherwise.

### A. Definitions

Unless defined otherwise, all technical and scientific terms used herein have the same meaning as commonly understood by one of ordinary skill in the art to which the invention pertains. Although a number of methods and materials similar or equivalent to those described herein can be used in the practice of the present invention, the preferred materials and methods are described herein.

In describing the present invention, the following terms will be employed, and are intended to be defined as indicated below.

The term "nucleic acid immunization" is used herein to refer to the introduction of a nucleic acid molecule encoding one or more selected antigens into a host cell for the *in vivo* expression of the antigen or antigens. The nucleic acid molecule can be introduced directly into the recipient subject, such as by injection; transdermal particle delivery; inhalation; topically, or by intranasal or mucosal modes of administration.

An "antigen" refers to any agent, generally a macromolecule, which can elicit an immunological response in an individual. The term may be used to refer to an individual macromolecule or to a homogeneous or heterogeneous population of antigenic macromolecules. As used herein, "antigen" is generally used to refer to a protein molecule or portion thereof which comprises one or more epitopes. For purposes of the present invention, antigens can be obtained or derived from any known virus, bacteria, parasite or fungal pathogen. The term also intends any of the various tumor-specific antigens and antigens associated with autoimmune diseases. Furthermore, for purposes of the present invention, an "antigen" includes a protein having modifications, such as deletions, additions and substitutions (generally conservative in nature) to the native sequence, so long as the protein maintains sufficient immunogenicity. These modifications may be deliberate, for example through site-directed mutagenesis, or may be accidental, such as through mutations of hosts which produce the antigens.

In various aspects of the invention, the antigen contains one or more T cell epitopes. A "T cell epitope" refers generally to those features of a peptide structure which are capable of inducing a T cell response. In this regard, it is accepted in the art that T cell epitopes comprise linear peptide determinants that assume extended conformations within the peptide-binding cleft of MHC molecules, (Unanue et al. (1987) *Science* 236:551-557). As used herein, a T cell epitope is generally a peptide having at least about 3-5 amino acid residues, and preferably at least 5-10 or more amino acid residues. The ability of a particular antigen to stimulate a cell-mediated immunological response may be determined by a number of well-known assays, such as by lymphoproliferation (lymphocyte activation) assays, CTL cytotoxic cell assays, or by assaying for T-lymphocytes specific for the antigen in a sensitized subject. See, e.g., Erickson et al. (1993) *J. Immunol.* 151:4189-4199; and Doe et al. (1994) *Eur. J. Immunol.* 24:2369-2376.

In other aspects of the invention, the antigen contains one or more B cell epitopes. A "B cell epitope" generally refers to the site on an antigen to which a specific antibody molecule binds. The identification of epitopes which are able to elicit an antibody response is readily accomplished using techniques well known in the art. See, e.g., Geysen et al. (1984) *Proc. Natl. Acad. Sci. USA* 81:3998-4002 (general method of rapidly synthesizing peptides to determine the location of immunogenic epitopes in a given antigen); U.S. Patent No. 4,708,871 (procedures for identifying and chemically synthesizing epitopes of antigens); and Geysen et al. (1986) *Molecular Immunology* 23:709-715 (technique for identifying peptides with high affinity for a given antibody).

An "immune response" against an antigen of interest is the development in an individual of a humoral and/or a cellular immune response to that antigen. For purposes of the present invention, a "humoral immune response" refers to an immune response mediated by antibody molecules, while a "cellular immune response" is one mediated by T-lymphocytes and/or other white blood cells.

A "coding sequence", or a sequence which "encodes" a polypeptide, is a nucleic acid molecule which is transcribed (in the case of DNA) and translated (in the case of mRNA) into a polypeptide *in vivo* when placed under the control of appropriate regulatory sequences. The boundaries of the coding sequence are determined by a start codon at the 5' (amino) terminus and a translation stop codon at the 3' (carboxy) terminus. For the purposes of the invention, a coding sequence can include, but is not limited to, cDNA from viral, procaryotic or eucaryotic mRNA, genomic DNA sequences from viral or procaryotic DNA, and even synthetic DNA sequences. A transcription termination sequence may be located 3' to the coding sequence.

A "nucleic acid" molecule can include, but is not limited to, procaryotic sequences, eucaryotic mRNA, cDNA from eucaryotic mRNA, genomic DNA sequences from eucaryotic (e.g., mammalian) DNA, and even synthetic DNA sequences. The term also captures sequences that include any of the known base analogs of DNA and RNA.

"Operably linked" refers to an arrangement of elements wherein the components so described are configured so as to perform their usual function. Thus, a given promoter operably linked to a coding sequence is capable of effecting the expression of the coding sequence when the proper enzymes are present. The promoter need not be contiguous with the coding sequence, so long as it functions to direct the expression thereof. Thus, for example, intervening untranslated yet transcribed sequences can be present between the promoter sequence and the coding sequence and the promoter sequence can still be considered "operably linked" to the coding sequence.

"Recombinant" as used herein to describe a nucleic acid molecule means a polynucleotide of genomic, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature.

Two nucleic acid sequences are "substantially homologous" when at least about 70%, preferably at least about 80-90%, and most preferably at least about 95%, of the nucleotides match over a defined length of the molecule. As used herein, substantially homologous also refers to sequences showing identity to the specified nucleic acid. Nucleic acid sequences that are substantially homologous can be identified in a Southern hybridization experiment under, for example, stringent conditions, as defined for that particular system. Defining appropriate hybridization conditions is within the skill of the art. See, e.g., Sambrook et al., *supra*; *DNA Cloning,* vols I & II, supra; *Nucleic Acid Hybridization, supra.* Such sequences can also be confirmed and further characterized by direct sequencing of PCR products.

The terms "individual" and "subject" are used interchangeably herein to refer to any member of the subphylum cordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The terms do not denote a particular age. Thus, both adult and newborn individuals are intended to be covered. The methods described herein are intended for use in any of the above vertebrate species, since the immune systems of all of these vertebrates operate similarly.

### B. General Methods

A minimal promoter is used in the present invention. The minimal promoter sequence is generally derived from a DNA virus. Typically, the promoter is a promoter which is associated with an early viral gene and usually modulated by an upstream or downstream enhancer element. In the present invention, however, the promoter sequence is used in its enhancerless form (i.e., it is not coupled with its native enhancer sequence when used in the context of the present invention, however, it may be used in a construct which contains other heterologous enhancer sequences). Thus, the minimal promoters of the present invention will typically be comprised of a nucleotide sequence of about 130 bases or less, which sequence will correspond to the sequence spanning positions -1 to about -130 relative to the start of RNA synthesis of the native viral early gene.

In preferred embodiments, the minimal promoter is obtained or derived from a member of the Herpesvirus family of viruses. In particular embodiments, the minimal promoter consists essentially of an enhancerless promoter sequence from a simian, murine or human CMV virus (for example, the sCMV or hCMV immediate early promoter), an enhancerless promoter sequence from a RSV virus, an enhancerless promoter sequence from a pseudorabies virus (PRV) such as the PRV early promoter region, or a functional variant thereof. The minimal promoter sequence may therefore consist essentially of a human cytomegalovirus (hCMV) immediate early promoter sequence, a pseudorabies virus (PRV) early promoter sequence or a functional variant thereof.

A functional variant sequence may vary from a native promoter sequence by one or more base substitutions, deletions or insertions. There may be from 1 to 30, for example from 5 to 20, base substitutions and/or from 1 to 30, for example from 5 to 20, base deletions and/or from 1 to 30, for example 5 to 20, base insertions. Functional fragments of a native promoter sequence may be used.

Variant sequences can readily be constructed by routine methodologies such as site-directed mutagenesis. The ability of a variant sequence to act as a promoter and thus retain function can be determined by experimentation. A variant sequence can be coupled to a reporter gene in a suitable expression system and the presence or absence of expression determined. The present invention is particularly applicable to humans, so the ability of a variant sequence to drive expression *in vitro* in a human cell line may be examined.

The minimal promoter sequence is coupled with a heterologous nucleotide sequence of interest, specifically a coding sequence for an antigen of a viral, bacterial, parasite or fungal pathogen, or a tumor-specific antigen or an antigen associated with an autoimmune disease. Thus, the minimal promoter will be operably linked to a coding sequence which encodes an antigen of interest. The antigen of interest will preferably be associated with a pathogen, such as a viral, bacterial or parasitic pathogen, or the antigen may be a tumor-specific antigen. The antigen may be a full length protein. Alternatively, the antigen may just consist essentially of a B-cell epitope or a T-cell epitope of an antigen.

Tumor-specific antigens include, but are not limited to, any of the various MAGEs (melanoma associated antigen E), including MAGE 1, MAGE 2, MAGE 3 (HLA-A1 peptide), MAGE 4, etc.; any of the various tyrosinases (HLA-A2 peptide); mutant ras; mutant p53; and p97 melanoma antigen. Other tumor-specific antigens include the Ras peptide and p53 peptide associated with advanced cancers, the HPV 16/18 and E6/E7 antigens associated with cervical cancers, MUC1-KLH antigen associated with breast carcinoma, CEA (carcinoembryonic antigen) associated with colorectal cancer, gp100 or MART1 antigens associated with melanoma, and the PSA antigen associated with prostate cancer. The *p53* gene sequence is known (see e.g., Harris et al. (1986) *Mol. Cell. Biol*. 6:4650-4656) and is deposited with GenBank under Accession No. M14694.

Suitable viral antigens include, but are not limited to, polynucleotide sequences encoding antigens from the hepatitis family of viruses, including hepatitis A virus (HAV), hepatitis B virus (HBV), hepatitis C virus (HCV), the delta hepatitis virus (HDV), hepatitis E virus (HEV) and hepatitis G virus (HGV). By way of example, the viral genomic sequence of HBV is known, as are methods for obtaining antigen-encoding sequences therefrom. See, e.g., Ganem et al. (1987) *Annu. Rev. Biochem.* 56:651-693; Hollinger, F.B. (1990) *Hepatitis B virus,* vol. II, pp.2171-2235, in Fields et al. (eds), Virology, 2nd ed, Raven Press, New York, NY; and Valenzuela et al. (1980) *The nucleotide Sequence of the Hepatitis B viral Genome and the Identification of the Major Viral Genes,* pp. 57-70, in Fields et al. (eds), Animal Virus Genetics, Academic Press, New York, NY). The HBV genome encodes several viral proteins, including the large, middle and major surface antigen polypeptides, the X-gene polypeptide, and the core polypeptide. See, e.g., Yokosuka et al. (1986) *N. Engl. J. Med.* 315:1187-1192; Imazeki et al. (1987) *Hepatology* 7:753-757; Kaneko et al. (1988) *J. Virol.* 62:3979-3984; and Ou et al. (1990) *J*. *Virol.* 64:4578-4581. In like manner, the viral genomic sequence of HCV is known, as are methods for obtaining the sequence. See, e.g., International Publication Nos. WO 89/04669; WO 90/11089; and WO 90/14436. The HCV genome encodes several viral proteins, including E1 and E2. See, e.g., Houghton et al. (1991) *Hepatology* 14:381-388. The sequences encoding these HBV and HCV proteins, as well as antigenic fragments thereof, will find use in the present methods. Similarly, the coding sequence for the δ-antigen from HDV is known (see, e.g., U.S. Patent No. 5,378,814).

In like manner, sequences encoding a wide variety of protein antigens from the herpesvirus family can be used in the present invention, including antigens derived from herpes simplex virus (HSV) types 1 and 2, such as HSV-1 and HSV-2 glycoproteins gB, gD and gH; antigens from varicella zoster virus (VZV), Epstein-Barr virus (EBV) and cytomegalovirus (CMV) including CMV gB and gH; and antigens from other human herpesviruses such as HHV6 and HHV7. (See, e.g. Chee et al. (1990) *Cytomegaloviruses* (J.K. McDougall, ed., Springer-Verlag, pp. 125-169; McGeoch et al. (1988) *J. Gen. Virol.* 69:1531-1574; U.S. Patent No. 5,171,568; Baer et al. (1984) *Nature* 310:207-211; and Davison et al. (1986) *J. Gen. Virol.* 67:1759-1816.)

HIV antigens, such as the gp120 sequences for a multitude of HIV-1 and HIV-2 isolates, including members of the various genetic subtypes of HIV, are known and reported (see, e.g., Myers et al., Los Alamos Database, Los Alamos National Laboratory, Los Alamos, New Mexico (1992); and Modrow et al. (1987) *J*. *Virol.* 61:570-578) and antigens derived from any of these isolates will find use in the present methods. Furthermore, the invention is equally applicable to other immunogenic moieties derived from any ,of the various HIV isolates, including any of the various envelope proteins such as gp160 and gp41, gag antigens such as p24gag and p55gag, as well as proteins derived from the *pol, env, tat, vif, rev, nef, vpr, vpu* and LTR regions of HIV.

Sequences encoding antigens derived or obtained from other viruses will also find use in the claimed methods, such as without limitation, sequences from members of the families Picornaviridae (e.g., polioviruses, etc.); Caliciviridae; Togaviridae (e.g., rubella virus, dengue virus, etc.); Flaviviridae; Coronaviridae; Reoviridae; Birnaviridae; Rhabodoviridae (e.g., rabies virus, etc.); Filoviridae; Paramyxoviridae (e.g., mumps virus, measles virus, respiratory syncytial virus, etc.); Bunyaviridae; Arenaviridae; Retroviradae (e.g., HTLV-I; HTLV-II; HIV-1 (also known as HTLV-III, LAV, ARV, hTLR, etc.)), including but not limited to antigens from the isolates HIV_{IIIb}, HIV_{SF2}, HIV_{LAV}, HIV_{LA1}, HIV_{MN}); HIV-1_{CM235}, HIV-1_{US4}; HIV-2, among others. See, e.g. Virology, 3rd Edition (W.K. Joklik ed. 1988); *Fundamental Virology,* 2nd Edition (B.N. Fields and D.M. Knipe, eds. 1991), for a description of these and other viruses.

Sequences encoding suitable bacterial and parasitic antigens are obtained or derived from known causative agents responsible for diseases such as Diptheria, Pertussis, Tetanus, Tuberculosis, Bacterial or Fungal Pneumonia, Cholera, Typhoid, Plague, Shigellosis or Salmonellosis, Legionaire's Disease, Lyme Disease, Leprosy, Malaria, Hookworm, Onchocerciasis, Schistosomiasis, Trypamasomialsis, Lesmaniasis, Giardia, Amoebiasis, Filariasis, Borelia, and Trichinosis. Still further antigens can be obtained or derived from unconventional viruses or virus-like agents such as the causative agents of kuru, Creutzfeldt-Jakob disease (CJD), scrapie, transmissible mink encephalopathy, and chronic wasting diseases, or from proteinaceous infectious particles such as prions that are associated with mad cow disease.

Both the sequence for the minimal promoter and the coding sequence of interest can be obtained and/or prepared using known methods. For example, substantially pure antigen preparations can be obtained using standard molecular biological tools. That is, polynucleotide sequences coding for the above-described antigens can be obtained using recombinant methods, such as by screening cDNA and genomic libraries from cells expressing the gene, or by deriving the gene from a vector known to include the same. Furthermore, the desired gene or promoter sequence can be isolated directly from cells and tissues containing the same, using standard techniques, such as phenol extraction and PCR of cDNA or genomic DNA. See, e.g., Sambrook et al., *supra,* for a description of techniques used to obtain and isolate DNA. Polynucleotide sequences can also be produced synthetically, rather than cloned.

Yet another convenient method for isolating specific nucleic acid molecules is by the polymerase chain reaction (PCR). Mullis et al. (1987) *Methods Enzymol*. 155:335-350. This technique uses DNA polymerase, usually a thermostable DNA polymerase, to replicate a desired region of DNA. The region of DNA to be replicated is identified by oligonucleotides of specified sequence complementary to opposite ends and opposite strands of the desired DNA to prime the replication reaction. The product of the first round of replication is itself a template for subsequent replication, thus repeated successive cycles of replication result in geometric amplification of the DNA fragment delimited by the primer pair used.

Once the sequences for the minimal promoter and the coding sequence of interest have been obtained, they are operably linked together to provide a nucleic acid molecule using standard cloning or molecular biology techniques. See, e.g., Edge (1981) *Nature* 292:756; Nambair *et al* (1984) *Science* 223:1299; and Jay *et al* 91984) *J. Biol. Chem*. 259:6311. The nucleic acid molecule is then inserted into a suitable vector such as an expression plasmid or viral vector construct.

The nucleic acid construct comprising the minimal promoter and a selected coding sequence generally also comprises other control sequences. These other control sequences typically comprise a terminator and/or translation initiation sequence (e.g. GCCACCATGG or GCCCCCATGG) and/or translational stop codon (e.g. TAA, TAG or TGA) and/or polyadenylation signal and/or a RNA pause site. The native enhancer for the promoter sequence is not present, however. Generally, no enhancer is present at all.

Once complete, the constructs are used for nucleic acid immunization using standard gene delivery protocols. Methods for gene delivery are known in the art. See, e.g., U.S. Patent Nos. 5,399,346, 5,580,859, 5,589,466. Genes can thus be delivered directly to a subject.

Liposomal preparations can be used to deliver the nucleic acid molecules of the invention. Useful liposomal preparations include cationic (positively charged), anionic (negatively charged) and neutral preparations, with cationic liposomes particularly preferred. Cationic liposomes have been shown to mediate intracellular delivery of plasmid DNA (Felgner et al. (1987) *Proc. Natl. Acad. Sci. USA* 84:7413-7416) and mRNA (Malone et al. (1989) *Proc. Natl. Acad. Sci. USA* 86:6077-6081).

Alternatively, the nucleic acid molecules of the present invention may be encapsulated, adsorbed to, or associated with, particulate carriers. Suitable particulate carriers include those derived from polymethyl methacrylate polymers, as well as PLG microparticles derived from poly(lactides) and poly(lactide-co-glycolides). See, e.g., Jeffery et al. (1993) Pharm. Res. 10:362-368. Other particulate systems and polymers can also be used, for example, polymers such as polylysine, polyarginine, polyomithine, spermine, spermidine, as well as conjugates of these molecules.

Formulation of a composition comprising the above nucleic acid molecules can be carried out using standard pharmaceutical formulation chemistries and methodologies all of which are readily available to the reasonably skilled artisan. For example, compositions containing one or more nucleic acid molecules can be combined with one or more pharmaceutically acceptable excipients or vehicles. Auxiliary substances, such as wetting or emulsifying agents, pH buffering substances and the like, may be present in the excipient or vehicle. These excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. Certain facilitators of nucleic acid uptake and/or expression can also be included in the compositions, for example, facilitators such as bupivacaine, cardiotoxin and sucrose. A thorough discussion of pharmaceutically acceptable excipients, vehicles and auxiliary substances is available in REMINGTON'S PHARMACEUTICAL SCIENCES (Mack Pub. Co., N.J. 1991), incorporated herein by reference.

When used in nucleic acid immunizations, the formulated compositions will include an amount of the antigen of interest which is sufficient to mount an immunological response, as defined above. An appropriate effective amount can be readily determined by one of skill in the art. Such an amount will fall in a relatively broad range that can be determined through routine trials. The compositions may contain from about 0.1% to about 99.9% of the antigen and can be administered directly to the subject using methods known to those skilled in the art. Methods for *in vivo* delivery can entail injection using a conventional syringe. The constructs can be injected either subcutaneously, epidermally, intradermally, intramucosally such as nasally, rectally and vaginally, intraperitoneally, intravenously, orally or intramuscularly. Other modes of administration include transdermal applications.

It is preferred, however, that the nucleic acid molecules be delivered using a particle acceleration device which fires nucleic acid-coated microparticles into target tissue, or transdermally delivers particulate nucleic acid compositions, In this regard, particle-mediated (sometimes referred to as "gene gun") nucleic acid immunization has been shown to elicit both humoral and cytotoxic T lymphocyte immune responses following epidermal delivery of nanogram quantities of DNA. Pertmer et al. (1995) *Vaccine* 13:1427-1430. Particle-mediated delivery techniques have been compared to other types of nucleic acid inoculation, and found markedly superior. Fynan et al. (1995) *Int. J. Immunopharmacology* 17:79-83, Fynan et al. (1993) *Proc. Natl. Acad. Sci. USA* 90:11478-11482, and Raz et al. (1994) *Proc. Natl. Acad. Sci. USA* 91:9519-9523. Such studies have investigated particle-mediated delivery of nucleic acid-based vaccines to both superficial skin and muscle tissue.

Particle-mediated methods for delivering nucleic acid preparations are known in the art. Thus, once prepared and suitably purified, the above-described nucleic acid molecules can be coated onto carrier particles using a variety of techniques known in the art. Carrier particles are selected from materials which have a suitable density in the range of particle sizes typically used for intracellular delivery from a gene gun device. The optimum carrier particle size will, of course, depend on the diameter of the target cells.

For the purposes of the invention, tungsten, gold, platinum and iridium carrier particles can be used. Tungsten and gold particles are preferred. Tungsten particles are readily available in average sizes of 0.5 to 2.0 µm in diameter. Gold particles or microcrystalline gold (e.g., gold powder A1570, available from Engelhard Corp., East Newark, NJ) will also find use with the present invention. Gold particles provide uniformity in size (available from Alpha Chemicals in particle sizes of 1-3 µm, or available from Degussa, South Plainfield, NJ in a range of particle sizes including 0.95 µm). Microcrystalline gold provides a diverse particle size distribution, typically in the range of 0.5-5 µm. However, the irregular surface area of microcrystalline gold provides for highly efficient coating with nucleic acids.

A number of methods are known and have been described for coating or precipitating DNA or RNA onto gold or tungsten particles. Most such methods generally combine a predetermined amount of gold or tungsten with plasmid DNA, CaCl₂ and spermidine. The resulting solution is vortexed continually during the coating procedure to ensure uniformity of the reaction mixture. After precipitation of the nucleic acid, the coated particles can be transferred to suitable membranes and allowed to dry prior to use, coated onto surfaces of a sample module or cassette, or loaded into a delivery cassette for use in particular gene gun instruments.

Various particle acceleration devices suitable for particle-mediated delivery are known in the art, and are all suited for use in the practice of the invention. Current device designs employ an explosive, electric or gaseous discharge to propel the coated carrier particles toward target cells. The coated carrier particles can themselves be releasably attached to a movable carrier sheet, or removably attached to a surface along which a gas stream passes, lifting the particles from the surface and accelerating them toward the target. An example of a gaseous discharge device is described in U.S. Patent No. 5,204,253. An explosive-type device is described in U.S. Patent No. 4,945,050. One example of a helium discharge-type particle acceleration apparatus is the PowderJect XR® instrument (PowderJect Vaccines, Inc., Madison), WI, which instrument is described in U.S. Patent No. 5,120,657. An electric discharge apparatus suitable for use herein is described in U.S. Patent No. 5,149,655. The disclosure of all of these patents is incorporated herein by reference.

The particulate nucleic acid compositions can alternatively be administered transdermally using a needleless syringe device. For example, a particulate composition comprising the nucleic acid constructs of the present invention can be obtained using general pharmaceutical methods such as simple evaporation (crystallization), vacuum drying, spray drying or lyophilization. If desired, the particles can be further densified using the techniques described in commonly owned International Publication No. WO 97/48485, incorporated herein by reference. These particulate compositions can then be delivered from a needleless syringe system such as those described in commonly owned International Publication Nos. WO 94/24263, WO 96/04947, WO 96/12513, and WO 96/20022, all of which are incorporated herein by reference.

The particle compositions or coated particles are administered to the individual in a manner compatible with the dosage formulation, and in an amount that will be effective for the purposes of the invention. The amount of the composition to be delivered depends on the individual to be tested. The exact amount necessary will vary depending on the age and general condition of the individual to be treated, and an appropriate effective amount can be readily determined by one of skill in the art upon reading the instant specification.

### C. Experimental

Below are examples of specific embodiments for carrying out the present invention. The examples are offered for illustrative purposes only, and are not intended to limit the scope of the present invention.

Efforts have been made to ensure accuracy with respect to numbers used (*e.g.,* amounts, temperatures, etc.), but some experimental error and deviation should, of course, be allowed for.

### Example 1

A number of expression systems were constructed to compare antigen expression from vectors containing an antigen coding sequence under transcriptional control of an enhanced promoter and the corresponding minimal promoter (using *in vitro* expression testing methods). The immunogenicity of the same vector constructs was then assessed (ability to elicit an antibody response against the antigen) using *in vivo* nucleic acid immunization testing methods.

Particularly, vectors containing the simian CMV promoter with ("sCMV") and without ("mP-sCMV") it enhancer, vectors containing the human CMV immediate early promoter with ("CMV") and without ("mp" or "mP-CMV") its enhancer, and vectors containing the PRV promoter with ("PRV") and without ("mP-PRV") its enhancer were assessed for both antigen production (*in vitro* expression) and for antibody production (*in vivo* immunogenicity). The results are depicted in the attached Figure 1. In all cases, removal of the enhancer sequences reduced expression levels from the minimal promoter constructs (relative to the enhanced promoters) (not shown). However, as can be seen in the figure, the minimal promoter constructs provided for dramatically increased antibody production (relative to the enhanced promoters) in the *in vivo* component of the study

### Example 2

In order to further assess the effect of using an enhancer-less promoter system in a nucleic acid immunization protocol, a number of experiments were carried out to compare the immunogenicity of antigen constructs containing full-length (enhanced) promoter systems and the minimal (enhancer-less) promoter systems of the present invention.

More particularly, a series of Hepatitis B surface antigen (HBsAg) constructs were constructed as follows. To generate the HbsAg coding region, the pAM6 construct (obtained from the American Type Culture Collection "ATCC") was cut with *Nco*1 and treated with mung bean nuclease to remove the start codon of the X-antigen. The resultant DNA was then cut with *Bam*H1 and treated with T4 DNA polymerase to blunt-end the DNA and create an HBsAg expression cassette. The HbsAg expression cassette is present in the 1.2 kB fragment. The plasmid construct PJV7077 (Schmaljohn et al. (1997) *J Virol.* 71:9563-9569) which contains the full-length human CNFV (Towne strain) immediate early promoter (with enhancer) was cut with Hind3 and *Bgl*2, and then treated with T4 DNA polymerase and calf-alkaline phosphatase to create blunt-ended DNA adjacent.

The PJV7232 host plasmid (a derivative of the PJV7077 construct described above) contains a promoter insertion region flanked by a *Sal*1 and *Bam*H1 site. New promoters were PCRer out of viral stocks, infected cells, plasmids, etc., using primers homologous to the promoter region of interest. These primers were designed with *Sal*1 and *Bam*H1 restriction sites at the terminal ends such that the PCR fragment can be cut with these enzymes to facilitate ready insertion into the similarly cut PJV7232 construct. This method was then used to construct HBsAg expression cassettes driven by full-length (enhanced) simian CMV (sCMV) and pseudorabies virus (PRV) promoter systems. All PCR reactions were carried out under standard conditions to amplify the promoter elements from the target sequences according to manufacturer instructions, particularly:
1x PCR core buffer w/ 15mM MgCl_{2;}
0.4 µM each of sense and antisense primers;
200 µM of each dNTP;
2.5 µg Taq polmerase;
0.1-1.0 µg target sample (ATCC# VR706 for sCMV and ATCC# VR135 for PRV);
water to 100 µL; and
mineral oil overlay.

the thermocycler was programmed to run the following routine:
4 minutes at 95°C;
30 cycles of (1 minute at 95° + 1 min.15 sec. at 55°C + 1 min. at 72°C);
10 minutes at 72°C; and
4°C hold.

After theromcycling was completed, the Taq polymerase was removed by phenol/chloroform extraction and ethanol precipitation of the PCR products. This amplified DNA, as well as the PJV7232 construct were then cut with the *Sal*1 and *Bam*H1 restriction enzymes.

After being cut with the appropriate restriction enzymes (and blunt-ended if needed), the DNAs were run on an agarose gel to separate, purify and isolate DNA bands of interest. These isolated bands were then mixed together in a ligase reaction and incubated overnight at 16°C. The ligation reactions were transformed into *E.coli* cells. The cut PJV7077 or PJV7232 constructs are not able to grow in the bacterial host cells under antibiotic selection unless they contain an appropriate insert fragment. Accordingly, bacterial colonies containing the new recombinant DNA constructs (the HbsAG sequence promoted by a full length hCMV, sCMV or PRV promoter) were grown on an agar plate, and the DNA was isolated from these colonies. The DNA was analysed for proper ligation of the various fragments. Bacterial isolates which harbor correct ligation products were amplified in a large liquid culture, on which a large-scale DNA extraction was performed. The resultant DNA is then used as a vaccine lot

In order to make corresponding minimal promoter (enhancer-less) versions of the above-described hCMV-sAG, sCMV-sAG and PRV-sAG constructs, the DNAs were cut with restriction enzyme pairs (*Sal*1/*Bam*1, *Sal*1/*Sca*1, or *Sal*1/*Not*1, respectively). The cut DNA was treated with T4 DNA polymerase to create blunt-ended DNA. The blunt-ended DNA was self-ligated, transformed, analyzed and amplified using the same conditions set forth herein above. This procedure produced vaccine lots were at least the majority of each enhancer sequence was deleted from the promoter.

The DNA vaccine products were then coatd onto gold carrier particles and administered to murine subjects using a particle-mediated delivery technique. Specifically, for each plasmid DNA construct to be tested, 25 mg of 2 µm gold powder was weighed into a microfuge tube. After addition of a 250 µL aliquot of 50 mM spermidine (Aldrich), the tubes were vortexed and briefly sonicated. The gold was then microfuged out, and the spermidine replaced by a fresh 100 µL aliquot. The gold was then resuspended by vortexing, after which 25 µg of DNA was added to the tubes and mixed. While the tube is lightly vortexed, 100 µL of 10% CaCl (Fujisawa) USA, Inc.) was added to precipitate the DNA onto the gold beads. The precipitation reaction was allowed to proceed for ten minutes on the benchtop, after which the gold was collected by a brief microfuge spin and washed three times with absolute ethanol (Spectrum) to remove excess precipitation reagents. The washed gold/DNA complex was then resuspended with 0.05 mg/mL polyvinylpyrrolidone (360 kD, Spectrum) in absolute ethanol. The resultant slurry was then injected into a TEFZEL® tube (McMaster-Carr) housed in a tube turner coating machine (PowderJect Vaccines, Inc., Madison WI) which coats the inside of the tube with the gold/DNA complex. This tube turner machine is described in US Patent No. 5,733,600. After the coating procedure was completed, the tubes were cut into 0.5 inch "shots" which were loaded into a particle-mediated delivery device (the PowderJect XR1 device, PowderJect Vaccines, Inc., Madison, WI) for delivery into the mice.

For the vaccination procedures, four- to six-week old Balb/c mice were anesthestized with a mixture of KETASET® (Fort Dodge) and ROMPUN® (Bayer) anestehtics. The bellies were shaved with a pair of electric clippers to remove hair, and two non-overlapping "shots" of vaccine were delivered from the PowderJect XR1 device (at 450 psi) to the shaved area. The animals were returned to their cages for six weeks after which time blood samples were obtained for sera analysis of anti-HBsAg antibodies.

More particularly, blood samples were harvested from the vaccinated animals. Up to 200 µL aliquots of serum isolated from the blood samples were then placed int wells of a reaction vessel supplied with the AUSAB® EIA Diagnostic Kit (Abbott Laboratories). The amount of sera added depended upon the antibody titer of the sample, and each sample was diluted with sample dilution buffer to fall within values that are readable by the immunoassay kit. 200 µL aliquots of standardization panel samples were added to the wells of the reaction vessel. A bead was added to each well, after which the vessel was sealed and incubated for two hours at 40°C. The wells were then washed of all liquid reaction components. 200 µL aliquots of the conjugate mix was then added to each washed well, after which the vessel was sealed and incubated for two hours again at 40°C. The wells were then washed of all liquid reaction components, and the bead transferred to new reaction tubes after which 300 µL of the color development buffer was added. After 30 minutes, the color development reaction was stopped by addition of 1M H₂SO₄, and the absorbance was measured at 490nm using a Quantum II™ spectrophotometer. The spectrophotometer was used to calculate antibody levels of each sample by comparison of the absorbance of the sample against a standard curve generated with a standardization panel. The antibody levels were then corrected for any dilution factors, and the final data reported as the geometric mean titers of all animals vaccinated with a particular DNA vaccine construct.

The results of these studies are depicted in Figures 2-4. Figure 2 shows a comparison between the fully enhanced hCMV promoter system and the minimal hCMV promoter system of the present invention. As can be seen, the minimal promoter system gave an approximately three-fold improvement in antibody titer over the fully enhanced promoter system (the results were pooled from four independent experiments of 8 mice per group). Figures 3 and 4 show similar results from comparisons between full-length (enhanced) promoters and their corresponding minimal (enhancer-less) promoter derivatives.

Accordingly, novel minimal promoter systems and nucleic acid reagents comprising these systems have been described. Although preferred embodiments of the subject invention have been described in some detail, it is understood that obvious variations can be made without departing from the scope of the invention as defined by the appended claims.

## Claims

1. Use of a nucleic acid construct comprising a coding sequence encoding an antigen and a promoter sequence which is not coupled to its native enhancer sequence and which is operably linked to the coding sequence, for the manufacture of a medicament for use in nucleic acid immunization of a mammalian subject, wherein said antigen is an antigen of a viral, bacterial, parasite or fungal pathogen, or is a tumor-specific antigen or an antigen associated with an autoimmune disease.

2. Use according to claim 1, wherein the antigen is a hepatitis B virus antigen,

3. Use according to claim 1 or 2, wherein the antigen is hepatitis B surface antigen.

4. Use according to claim 1, wherein the antigen is a HIV antigen.

5. Use according to any one of the preceding claims, wherein the antigen comprises a B-cell epitope or a T-cell epitope

6. Use according to any one of the preceding claims, wherein the construct is delivered directly into the said subject.

7. Use according to claim 6, wherein the construct is delivered by injection, transdermal particle delivery, inhalation, topically, intranasally or transmucosally.

8. Use according to claim 7, wherein the construct is delivered by needleless injection.

9. Use according to any one of the preceding claims, wherein the construct is coated onto carrier particles for delivery of the construct to the said subject.

10. Use according to claim 9, wherein the carrier particles are tungsten or gold particles.

11. Use according to any one of the preceding claims, wherein the said subject is a human.

12. Use according to any one of the preceding claims, wherein the construct is a DNA construct.

13. Use according to any one of the preceding claims, wherein the promoter sequence consists essentially of a human cytomegalovirus (hCMV) immediate early promoter sequence, a pseudorabies virus (PRV) early promoter region, a simian cytomegalovirus (sCMV) immediate early promoter sequence or a functional variant thereof

14. Use according to claim 13, wherein the promoter sequence consists essentially of the sequence spanning positions 0 to -118 of the hCMV immediate early promoter region or a functional variant of the said spanning sequence.

15. Coated particles suitable for use in particle-mediated nucleic acid immunisation, which particles comprise carrier particles coated with a nucleic acid construct comprising a coding sequence encoding an antigen and a promoter sequence which is not coupled to its native enhancer sequence and which is operably linked to the coding sequence, wherein said antigen is an antigen of a viral, bacterial, parasite or fungal pathogen, or is a tumor-specific antigen or an antigen associated with an autoimmune disease.

16. Coated particles according to claim 15, wherein the carrier particles are tungsten or gold particles.

17. Coated particles according to claim 15 or 16, wherein the antigen is defined in any one of claims 2 to 5.

18. Coated particles according to any one of claims 15 to 17, wherein the construct is a DNA construct.

19. Coated particles according to any one of claims 15 to 18, wherein the promoter sequence is as defined in claim 13 or 14.

20. A particle acceleration device suitable for particle-mediated nucleic acid immunization, the said device being loaded with coated particles as defined in any one of claims 15 to 19.

## Patentansprüche

1. , Verwendung eines Nukleinsäure-Konstrukts, umfassend eine codierende Sequenz, die ein Antigen codiert, und eine Promotorsequenz, die nicht an ihre native Enhancer-Sequenz gekoppelt ist und welche in funktionsfähiger Weise an die codierende Sequenz gebunden ist, zur Herstellung eines Medikaments zur Verwendung bei der Nukleinsäure-Immunisierung eines Säuger-Subjektes, wobei das Antigen ein Antigen eines viralen, bakteriellen, parasitären oder pilzlichen Pathogens ist, oder ein Tumor-spezifisches Antigen oder ein mit einer Autoimmunerkrankung assoziiertes Antigen ist.

2. Verwendung gemäß Anspruch 1, wobei das Antigen ein Hepatitis B Virus-Antigen ist.

3. Verwendung gemäß Anspruch 1 oder 2, wobei das Antigen Hepatitis B-Oberflächen-Antigen ist.

4. Verwendung gemäß Anspruch 1, wobei das Antigen ein HIV-Antigen ist.

5. Verwendung gemäß mindestens einem der vorhergehenden Ansprüche, wobei das Antigen ein B-Zell-Epitop oder ein T-Zell-Epitop umfasst.

6. Verwendung gemäß mindestens einem der vorhergehenden Ansprüche, wobei das Konstrukt direkt dem Subjekt zugeführt wird.

7. Verwendung gemäß Anspruch 6, wobei das Konstrukt durch Injektion, transdermale Telichenzuführung, Inhalation, topisch, intranasal oder transmukosal zugeführt wird.

8. Verwendung gemäß Anspruch 7, wobei das Konstrukt durch nadelfreie Injektion zugeführt wird.

9. Verwendung gemäß mindestens einem der vorhergehenden Ansprüche, wobei das Konstrukt auf Trägerteilchen zur Zuführung des Konstruktes zu dem Subjekt aufbeschichtet ist.

10. Verwendung gemäß Anspruch 9, wobei die Trägerteilchen Wolfram- oder Goldteilchen sind.

11. Verwendung gemäß mindestens einem der vorhergehenden Ansprüche, wobei das Subjekt ein Mensch ist.

12. Verwendung gemäß mindestens einem der vorhergehenden Ansprüche, wobei das Konstrukt ein DNA-Konstrukt ist.

13. Verwendung gemäß mindestens einem der vorhergehenden Ansprüche, wobei die Promotorsequenz im Wesentlichen aus einer Immediate-Early-Promotorsequenz vom humanen Cytomegalovirus (hCMV), einer Early-Promotorregion vom Pseudorabiesvirus (PRV), einer Immediate-Early-Promotorsequenz vom Simian-Cytomegalovirus (sCMV) oder einer funktionellen Variante davon besteht.

14. Verwendung gemäß Anspruch 13, wobei die Promotorsequenz im Wesentlichen aus der Sequenz, welche sich über die Positionen 0 bis -118 der Immediate-Early-Promotorregion vom hCMV erstreckt, oder einer funktionellen Variante dieser sich darüber erstreckenden Sequenz besteht.

15. Beschichtete Teilchen, die für die Verwendung in einer Teilchen-vermittelten Nukleininsäure-Immunisierung geeignet sind, wobei die Teilchen Trägerteilchen umfassen, die mit einem Nukleinsäurekonstrukt, das eine codierende Sequenz, die ein Antigen codiert, und eine Promotorsequenz, die nicht an ihre native Enhancer-Sequenz gekoppelt ist und welche in funktionsfähiger Weise an die codierende Sequenz gebunden ist, umfasst, wobei das Antigen ein Antigen eines viralen, bakteriellen, parasitären oder pilzlichen Pathogens ist, oder ein Tumor-spezifisches Antigen oder ein mit einer Autoimmunerkrankung assoziiertes Antigen ist, beschichtet sind.

16. Beschichtete Teilchen gemäß Anspruch 15, wobei die Trägerteilchen Wolfram- oder Goldteilchen sind.

17. Beschichtete Teilchen gemäß Anspruch 15 oder 16, wobei das Antigen in einem beliebigen der Ansprüche 2 bis 5 definiert ist.

18. Beschichtete Teilchen gemäß mindestens einem der Ansprüche 15 bis 17, wobei das Konstrukt ein DNA-Konstrukt ist.

19. Beschichtete Teilchen gemäß mindestens einem der Ansprüche 15 bis 18, wobei die Promotorsequenz wie in Anspruch 13 oder 14 definiert ist.

20. Teilchenbeschleunigungs-Vorrichtung, die zur Teilchen-vermittelten Nukleinsäure-Immunisierung geeignet ist, wobei die Vorrichtung mit beschichteten Teilchen, wie sie in mindestens einem der Ansprüche 15 bis 19 definiert sind, beladen wird.

## Revendications

1. Utilisation d'une construction d'acide nucléique comprenant une séquence codante codant un antigène et une séquence de promoteur qui n'est pas couplée à sa séquence d'activateur native et qui est liée de façon opérationnelle à la séquence codante, pour la fabrication d'un médicament destiné à être utilisé dans une immunisation par un acide nucléique d'un sujet mammifère, dans laquelle ledit antigène est un antigène d'un pathogène viral, bactérien, parasitaire ou fongique, ou est un antigène spécifique d'une tumeur ou un antigène associé à une maladie auto-immune.

2. Utilisation selon la revendication 1, dans laquelle l'antigène est un antigène de virus de l'hépatite B.

3. Utilisation selon la revendication 1 ou 2, dans laquelle l'antigène est un antigène de surface d'hépatite B.

4. Utilisation selon la revendication 1, dans laquelle l'antigène est un antigène du VIH.

5. Utilisation selon l'une quelconque des revendications précédentes, dans laquelle l'antigène comprend un épitope de cellule B ou un épitope de cellule T.

6. Utilisation selon l'une quelconque des revendications précédentes, dans laquelle la construction est directement délivrée dans ledit sujet.

7. Utilisation selon la revendication 6, dans laquelle la construction est délivrée par injection, délivrance transdermique de particules, inhalation, voie topique, intranasale ou transmuqueuse.

8. Utilisation selon la revendication 7, dans laquelle la construction est délivrée par injection sans aiguille.

9. Utilisation selon l'une quelconque des revendications précédentes, dans laquelle la construction est déposée sur des particules supports pour la délivrance de la construction audit sujet.

10. Utilisation selon la revendication 9, dans laquelle les particules supports sont des particules de tungstène ou d'or.

11. Utilisation selon l'une quelconque des revendications précédentes, dans laquelle ledit sujet est un humain.

12. Utilisation selon l'une quelconque des revendications précédentes, dans laquelle la construction est une construction d'ADN.

13. Utilisation selon l'une quelconque des revendications précédentes, dans laquelle la séquence de promoteur est essentiellement constituée d'une séquence de promoteur précoce immédiat de cytomégalovirus humain (hCMV), une région de promoteur précoce de virus de la pseudorage (PRV), une séquence de promoteur précoce immédiat de cytomégalovirus simien (sCMV) ou l'un de leurs variants fonctionnels.

14. Utilisation selon la revendication 13, dans laquelle la séquence de promoteur est essentiellement constituée de la séquence s'étendant des positions 0 à -118 de la région de promoteur précoce immédiat de hCMV ou d'un variant fonctionnel de ladite séquence ainsi étendue.

15. Particules revêtues appropriées à une utilisation dans une immunisation par acide nucléique médiée par des particules, ces particules comprennent des particules supports revêtues d'une construction d'acide nucléique comprenant une séquence codante codant un antigène et une séquence de promoteur qui n'est pas couplée à sa séquence d'activateur native et qui est liée de façon opérationnelle à la séquence codante, dans lesquelles ledit antigène est un antigène d'un pathogène viral, bactérien, parasitaire ou fongique, ou est un antigène spécifique d'une tumeur ou un antigène associé avec une maladie auto-immune.

16. Particules revêtues selon la revendication 15, dans lesquelles les particules supports sont des particules de tungstène ou d'or.

17. Particules revêtues selon la revendication 15 ou 16, dans lesquelles l'antigène est défini dans l'une quelconque des revendications 2 à 5.

18. Particules revêtues selon l'une quelconque des revendications 15 à 17, dans lesquelles la construction est une construction d'ADN.

19. Particules revêtues selon l'une quelconque des revendications 15 à 18, dans lesquelles la séquence de promoteur est telle que définie dans la revendication 13 ou 14.

20. Dispositif d'accélération de particules approprié à une immunisation par un acide nucléique médiée par des particules, ledit dispositif étant chargé avec des particules revêtues telles que définies dans l'une quelconque des revendications 15 à 19.
